# EUROPEAN PATENT SPECIFICATION

(11) **EP 4 181 933 B1**
(45) Date of publication and mention of the grant of the patent: **17.04.2024**
(21) Application number: 21742124.7
(22) Date of filing: 09.07.2021
(51) Int. Cl.: A61K 31/731, A61P 31/14

(54) **ANTIVIRAL PHARMACEUTICAL COMPOSITION COMPRISING AT LEAST ONE SULPHATED POLYSACCHARIDE**
ANTIVIRALE PHARMAZEUTISCHE ZUSAMMENSETZUNG ENTHALTEND MINDESTENS EIN SULFATIERTES POLYSACCHARID
COMPOSITION PHARMACEUTIQUE ANTIVIRALE COMPRENANT AU MOINS UN POLYSACCHARIDE SULFATÉ

(30) Priority: 16.07.2020 EP 20186334
(43) Date of publication of application: 24.05.2023
(73) Proprietor: Marinomed Biotech AG, 2100 Korneuburg (AT)
(72) Inventor: PRIESCHL-GRASSAUER, Eva, 1210 Wien (AT); GRASSAUER, Andreas, 1210 Wien (AT); MOROKUTTI-KURZ, Martina, 1130 Wien (AT); GRAF, Philipp, 2100 Korneuburg (AT)
(74) Representative: Bogensberger, Burkhard
(86) International application number: PCT/EP2021/069238
(87) International publication number: WO 2022/013113

(56) References cited:
- EP-A1- 1 930 015
- WO-A2-02/02189
- US-A1- 2005 004 071
- US-A1- 2012 237 572
- US-A1- 2019 060 358
- BABA M ET AL: "SULFATED POLYSACCHARIDES ARE POTENT AND SELECTIVE INHIBITORS OF VARIOUS ENVELOPED VIRUSES INCLUDING HERPES SIMPLEX VIRUS CYTOMEGALOVIRUS VESICULAR STOMATITIS VIRUS AND HUMAN IMMUNODEFICIENCY VIRUS", ANTIMICROBIAL AGENTS AND CHEMOTHERAPY, AMERICAN SOCIETY FOR MICROBIOLOGY, US, vol. 32, no. 11, 1 January 1988 (1988-01-01), pages 1742-1745, XP002413849, ISSN: 0066-4804
- LEIBBRANDT ANDREAS ET AL: "Iota-Carrageenan Is a Potent Inhibitor of Influenza A Virus Infection", PLOS ONE, PUBLIC LIBRARY OF SCIENCE, US, vol. 5, no. 12, 1 December 2010 (2010-12-01), pages e14320-1, XP009159153, ISSN: 1932-6203, DOI: 10.1371/JOURNAL.PONE.0014320 [retrieved on 2010-12-14]
- GONZALEZ M E ET AL: "POLYSACCHARIDES AS ANTIVIRAL AGENTS: ANTIVIRAL ACTIVITY OF CARRAGEENAN", ANTIMICROBIAL AGENTS AND CHEMOTHERAPY, AMERICAN SOCIETY FOR MICROBIOLOGY, US, vol. 31, no. 9, 1 September 1987 (1987-09-01), pages 1388-1393, XP000869535, ISSN: 0066-4804

## Description

### FIELD OF TECHNOLOGY

The present invention is in the fields of biochemistry, virology and medicine and relates to sulphated polysaccharides, and particularly to carrageenans, in the manufacture of an antiviral pharmaceutical composition or medicament for the prophylactic or therapeutic treatment of SARS-CoV-2-infection.

### BACKGROUND

The coronaviridae family includes coronavirus and torovirus. Coronaviruses are known to infect the upper respiratory tract and the gastrointestinal tract in mammals and birds. It is believed that coronaviruses cause a high percentage of all common colds in human adults.

WO2009/027057 discloses the use of fucoidans and carrageenans in the prophylactic and therapeutic treatment of various respiratory viral infections caused by members of the paramyxoviridae, orthomyxoviridae, adenoviridae and/or coronaviridae families. More precisely, WO2009/027057 *inter alia* reports the results of a coronavirus induced cell death inhibition experiment in cat kidney (CK) cells wherein the CK cells were infected with feline coronavirus FIP in the presence or absence of iota-, kappa- or lambda-carrageenan. It is further disclosed therein that all three types of carrageenan (i.e. iota-, kappa- and lambda-carrageenan) inhibit the coronavirus mediated cell death in CK cells at the highest concentration of 400 *µ*g/ml and that iota-carrageenan showed significant inhibition even at a concentration as low as 4 *µ*g/m), while kappa- and lambda-carrageenan were not effective at this concentration. It was concluded from the results that the carrageenan inhibitory effect on host cell penetration by corona virus was due to reasons other than a chemical or structural modification of the coronavirus-specific receptor(s). It was also found that the pretreatment of the host cells with carrageenan prior to infection did not significantly improve cell protection against coronavirus infection. The results suggested that in order to achieve a significant protection against coronavirus infection the carrageenan component acting as an antiviral agent must be present at the time of infection, i.e. when an interaction between the virus and the host cell is about to occur.

Sulphated polysaccharides including carrageenans and fucoidan have been known for their antiviral efficacy for decades (US2019/060358 A1; US 2012/237572 A1; US 2005/004071 A1). In a most interesting review, Gonzalez M.E. et al. (1987, Antimicrob. Agents Chemother. 31, 1388-1393) report an antiviral efficacy of different sulphated polysaccharides including iota-carrageenan against several animal viruses. Iota-carrageenan showed antiviral activity against the enveloped viruses HSV-1, HSV-2, Semliki Forest virus (SFV), vaccinia virus and African swine fever virus (ASF) and against the naked encephalomyocarditis (EMC) virus. Iota-carrageenan had no effect on the enveloped viruses vesicular stomatitis virus (VSV) and measles virus and on the naked viruses polio virus type 1 and adenovirus type 5.

In view of differing experiences made and further in view of sometimes even contradictory results obtained by researches in their attempts of antiviral treatment of varying viral species with carrageenan the authors of US 5,658,893 summarized at column 2, last paragraph bridging over to col. 3, that *"in view of the different responses by different viruses to sulphated polysaccharides, it is clear that the response of a particular virus to carrageenan cannot be predicted with certainty without experimentation. The mechanism by which sulphated polysaccharides, particularly the carrageenans, inhibit viral replication and infectivity may not be uniform as different investigators reported contradictory findings when working with different viruses and cell types. It would **not** be obvious to one skilled in the art that a substance such as a sulphated polysaccharide that is an effective inhibitor of one virus would demonstrate similar efficacy against another virus. "*

This situation has not changed over the years and is still applicable.

### BRIEF DESCRIPTION OF THE INVENTION

The challenging worldwide spreading pandemic coronavirus disease 2019 (COVID-19) is caused by a virus that has been denominated Severe Acute Respiratory Syndrome Coronavirus 2 (SARS-CoV-2). Before entering the cell the virus attaches to the host cell surface. It has been found that the viral spike protein recognizes and attaches to the ACE2-receptor (Angiotensin-Converting Enzyme 2 receptor) located on the surface of type I and II pneumocytes, endothelial cells, and ciliated bronchial epithelial cells. It is therefore very likely that drugs interfering with the interaction between the spike protein of SARS-CoV-2 and the ACE2 receptor may offer protection against infection by this kind of virus.

Based on this recognition of the pertinent art and further in view of own indepth knowledge of the characteristics and antiviral efficacy of carrageenans the present inventors have undertaken to find out whether carrageenan could also be effectively used in the prophylactic or therapeutic intervention of SARS-CoV-2 infections in mammals.

In order to accomplish this goal a suitable *in vitro* testing setup was used for infecting eukaryotic target cells with a commercially available modified lentivirus mimicking part of the outer surface of SARS-CoV2 and further bearing a luciferase reporter gene construct for facilitating analytical determination of infection rates.

It is an objective of the present invention to provide a useful antiviral composition or medicament comprising one or more specifically selected sulphated polysaccharides, for the prophylactic intervention of an infection, i.e. for reducing the risk of infection, of mammals including humans and non-human animals, by SARS-CoV-2.

It is a further objective of the present invention to provide a useful antiviral composition or medicament comprising one or more specifically selected sulphated polysaccharides, for the therapeutic treatment of SARS-CoV-2 infected individuals.

It is particularly envisaged to provide useful medication for individuals especially susceptible to or at increased risk of SARS-CoV-2 infection including high-risk patients selected from the group consisting of COPD-patients, asthma patients, individuals suffering from allergies, or from impaired immune systems, from impaired cardiac systems, or from impaired pulmonary systems, and transplantation patients, as well as healthcare personell including employees in hospitals, pharmacies and doctor's offices, and other persons who occupationally get into close contact with infected or potentially infected human individuals or non-human animals.

### FIGURES

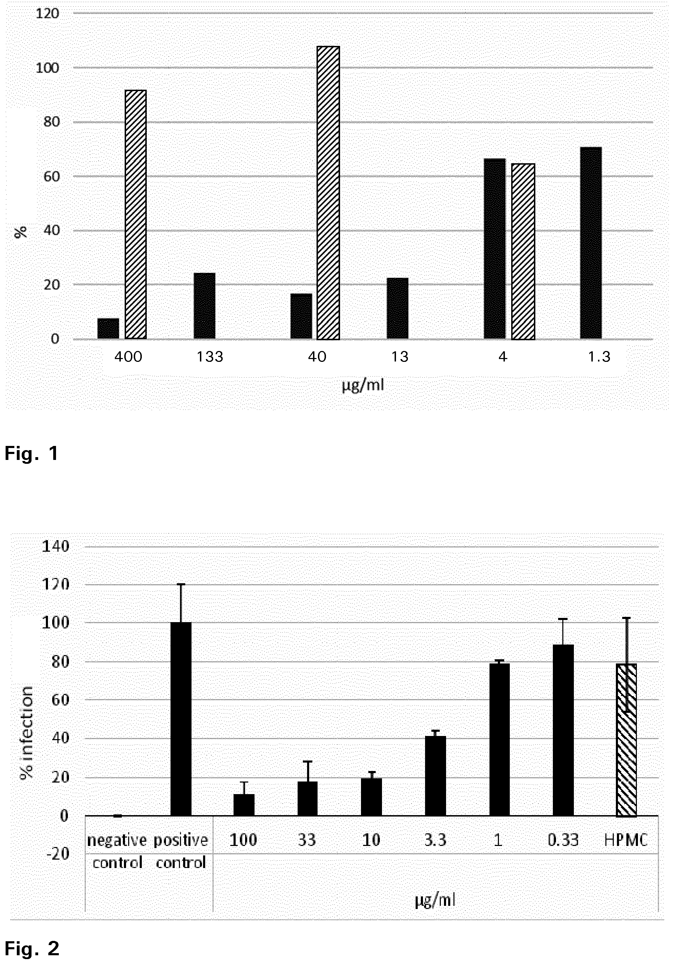

- Fig. 1: discloses the results of a neutralization assay demonstrating the inhibitory efficacy of iota-carrageenan and CMC at varying concentrations on the infection of ACE2-HEK293 cells with SARS-CoV-2 spike glycoprotein pseudotyped lentivirus bearing a luciferase reporter gene; y-axis = % infected cells relative to positive control (positive control = 100%; number of infected cells without carrageenan or CMC treatment); x-axis = varying concentrations of iota-carrageenan and CMC in ug/ml; black bars = iota carrageenan; hatched bars = CMC.
- Fig. 2: discloses the results of a neutralization assay demonstrating the inhibitory efficacy of iota-carrageenan and HPMC at varying concentrations on the infection of ACE2-HEK293 cells with SARS-CoV-2 spike glycoprotein pseudotyped lentivirus bearing a luciferase reporter gene; y-axis = % infected cells relative to positive control (positive control = 100%; number of infected cells without carrageenan or CMC treatment) and negative control (=0% infection; mock-infected cells); x-axis = varying concentrations of iota-carrageenan and HPMC in ug/ml; black bars = iota carrageenan; hatched bar = HPMC at 100 ug/ml.

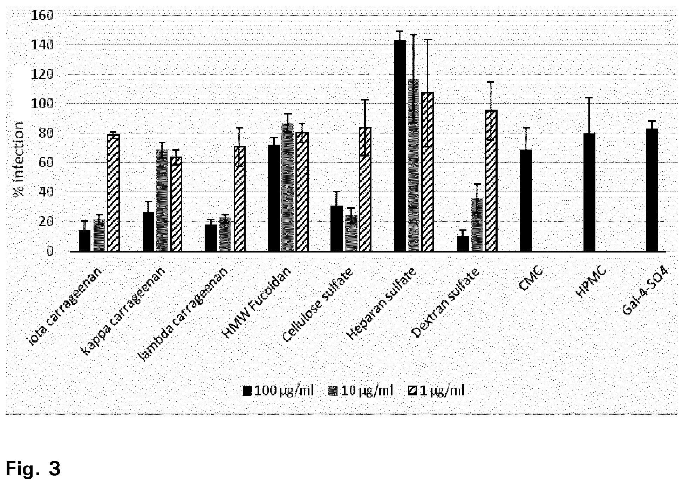

- Fig. 3: discloses the results of a neutralization assay demonstrating the inhibitory efficacy of various sulphated polymers at varying concentrations on the infection of ACE2-HEK293 cells with SARS-CoV-2 spike glycoprotein pseudotyped lentivirus bearing a luciferase reporter gene; y-axis = % infected cells relative to positive control (positive control = 100%; number of infected cells without carrageenan or CMC treatment); x-axis = various polymers tested at 3 different concentrations, i.e. 100ug/ml (black bars); 10ug/ml (grey bars); 1 ug/ml (hatched bars); CMC, HPMC and Gal-4-SO4 at 100ug/ml.

### DETAILED DESCRIPTION OF THE INVENTION

The term "antiviral active ingredient" as used herein refers to a compound that is effective in directly or indirectly interfering with at least one viral action selected from the group consisting of virus penetration of eukaryotic cells, virus replication in eukaryotic cells, virus assembly, virus release from infected eukaryotic cells, or that is effective in unspecifically inhibiting a virus titer increase or in unspecifically reducing a virus titer level in a eukaryotic or mammalian host system. It also refers to a compound that prevents an individual from or reduces an individual's risk or likelihood of catching a viral infection.

The present pharmaceutical composition may be administered, as the case may be, before or after the onset of a viral infection, i.e. for prophylactic or therapeutic treatment purposes, or for both prophylactic and therapeutic administration.

The terms "prophylaxis", "prophylactic intervention" or "prophylactic treatment" as used herein relate to the administration of the present pharmaceutical composition to a symptom-free, typically healthy, individual in order to reduce said individual's susceptibility for a viral infection with a SARS coronavirus.

The term "therapy", "therapeutic intervention" or "therapeutic treatment" as used herein relates to the administration of the present pharmaceutical composition to a human or non-human animal in order to achieve a reduction in severity and/or frequency of symptoms, elimination of symptoms and/or underlying cause, prevention of the occurrence of symptoms and/or their underlying cause, and/or improvement or remediation of damage directly caused by or indirectly associated, e.g. through secondary infection, with a viral infection by a SARS coronavirus, especially by SARS-CoV-2.

It is known that orthomyxo- and paramyxoviruses and several other viruses attach to the host cell via binding to receptors present on the host cell surface, the receptors typically being glycoproteins or glycolipids containing sugar residues including sialic acid (N-acetyl neuramic acid) and heparan sulphate residues.

In earlier *in vitro* studies it was observed by the present inventors that exposing host cells to carrageenan, in particular iota- and/or kappa-carrageenan, prior to infection may alter the structure of the cell surface of a said host cell, presumably by causing conformational changes in the three dimensional structure of certain cell surface receptors or by masking and/or modifying said receptors. Yet, a protective effect against coronavirus infection, including SARS-CoV-2 infection, was only achieved in the presence of carrageenan. It was therefore concluded that with coronavirus the antiviral effect may be due to attachment of the carrageenan polymer to the virions rather than to chemical or physical interaction of the polymer with the host cell receptors.

It seems that once carrageenan is administered to the site of infection at an antiviral effective concentration, it covers the viral surface by rather indiscriminate electrostatic interactions, thereby preventing the virus from binding to the mucosa and, as a result thereof, reducing its capability of causing a primary infection. In case that infection has already taken place, newly synthesized virus particles that are excreted from the cells are neutralized by the carrageenan polymers as well, resulting in an inhibition of viral spread. When applied as a nasal or mouth spray, the mean residence time of iota-, kappa-, or lambda-carrageenan at the mucosa is within a range of from 1 to 6 hours, and typically about three to four hours.

Carrageenan is a generic term for linear sulphated galactose-based polysaccharides extracted from red seaweed (rhodophyceae). There exist more than 10 structurally different carrageenans, their nature depending on the seaweed genus from which they are extracted. The three main types are iota-, kappa- and lambda-carrageenan, which differ slightly in their structure and in the degree of sulphatation. Iota-carrageenan is a soft-gel forming sulphated galactan predominantly extracted from the red seaweed *Eucheuma denticulatum (syn. Eucheuma spinosum).* Kappa-carrageenan yields strong, rigid gels and is predominantly obtained from *Kappaphycus alvarezii (syn. Eucheuma cottonii).* Lambda-carrageenan, typically obtained from the tetrasporophytes of Chondrus crispus, is frequently used to thicken dairy products.

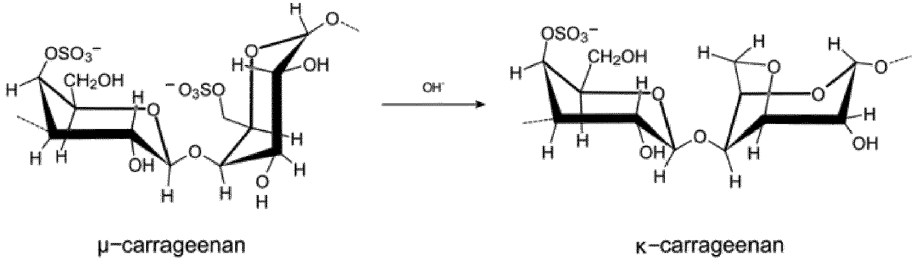

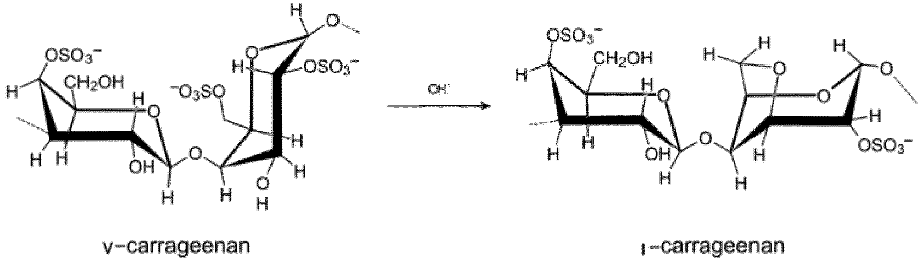

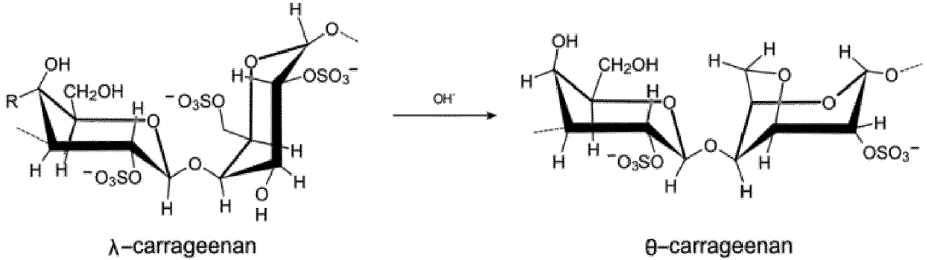

The commercially available carrageenans used in the present invention are typically mixtures of hybrid or heteropolymer molecules of different chain lengths and molecular weights. Carrageenans useful for topical application to the mucosa in accordance with the present invention have a molecular weight ranging from about 15,000 to 5,000,000 Da (15 kDa - 5 MDa), wherein fractions having average molecular weights of more than 50,000 Da (50 kDa), and especially fractions having average molecular weights in the range of from 50,000 to 3,000,000 Da (50 kDa - 3 MDa) are particularly preferred.

The carrageenan-type sulphated polysaccharides comprise a linear backbone made up of repeating galactose-based disaccharide subunits of either prevailingly the iota- or prevailingly the kappa-type disaccharide subunits. In case of commercially available lambda-type carrageenan, the linear polymer backbone further comprises substantial amounts of lambda-type subunits, along with kappa- and/or iota-type disaccharide subunits. Carrageenan homopolymers built of subunits of only one kind of either iota-, or kappa-, or lambda-type, are not usually observed in nature. However, depending on the nature of the selected red seaweed source and the kind of extraction of the carrageenan content, starting materials having a purity of up to 95% of either iota-type or kappa-type subunits within the polymer backbone are obtainable.

For the purpose of the present invention the term "iota-carrageenan" shall refer to a sulphated polysaccharide of the carrageenan type comprising in its linear backbone more than 50 mol%, typically more than 75 mol% and preferably at least 90 mol% of iota-type disaccharide repeating subunits, including any amounts of its precursor "nu-type" subunits that may simultaneously be present in the iota-carrageenan starting material, as determined by 1H-NMR spectroscopy or by any other suitable analytical method known in the art. The remaining repeating disaccharide subunits mainly being of the kappa-tpye. In the pertinent literature this type of carrageenan is sometimes also referred to as iota/kappa-carrageenan, as opposed to kappa/iota-carrageenan.

Analogously, for the purpose of the present invention the term "kappa-carrageenan" shall refer to a sulphated polysaccharide of the carrageenan type comprising in its linear backbone more than 50 mol%, typically more than 75 mol% and preferably at least 90 mol% of kappa-type disaccharide repeating subunits, including any amounts of its precursor "mu-type" subunits that may simultaneously be present in the kappa-carrageenan starting material, as determined by 1H-NMR spectroscopy or any other suitable analytical method known in the art. The remaining repeating disaccharide subunits mainly being of the iota-tpye. In the pertinent literature this type of carrageenan is sometimes also referred to as kappa/iota-carrageenan, as opposed to iota/kappa-carrageenan.

The term lambda-carrageenan shall refer to a sulphated polysaccharide of the carrageenan type comprising in its linear backbone at least 5 mol%, typically at least 10 mol% and preferably at least 20 mol% of lambda-type disaccharide repeating subunits, the remaining repeating disaccharide subunits typically being mainly of the kappa-type and/or iota-type, as determined by 1H-NMR spectroscopy or any other suitable analytical method known in the art.

Accordingly, commercially available "iota-carrageenan" material useful in the present invention mainly comprises iota/kappa-type hybrid polymers, whereas commercially available "kappa-carrageenan"material useful for the present invention typically comprises mainly kappa/iota hybrid polymers, and the commercially available "lambda carrageenans" useful in the present invention mainly comprise kappa/iota-type hybrid polymers along with a substantial share of kappa/lambda and/or kappa/iota/lambda-, and/or iota/kappa/lambda-type hybrid polymers. It may, however, be advantageous to purify the commercially available lambda-carrageenan material in order to increase the proportion of the lambda-type repeating disaccharide subunits relative to the remaining kappa-, iota-, mu- and nu-type subunits present in the lambda-carrageenan starting material, and in doing so to increase its antiviral potential and effectiveness.

A "mixture" of carrageenans in the sense of the present invention therefore refers to a composition of matter comprising a mixture of at least two of iota-, kappa-, and lambda-type carrageenans as defined hereinbefore, i.e. to a mixture of at least two of iota/kappa-, kappa/iota-, kappa/lambda-, kappa/iota/lambda-, and iota/kappa/lambda-type hybrid carrageenans.

Typically, the antiviral pharmaceutical compositions according to the present invention are substantially free of carrageenans other than iota-, kappa-, lambda-, mu- and nu-type carrageenan.

Fucoidan is another sulphated polysaccharide mainly extracted from various species of brown seaweed, typically from *Undaria pinnatifida.* There are two types of pharmacological grade fucoidans available on the market, a high molecular weight fucoidan (HMWF) fraction having an average molecular weight ranging from about 1,000,000 to 2,000,000 Da (e.g. Kraeber, Germany) and a low molecular weight fucoidan (LMWF) fraction having an average molecular weight of 8,200 Da. F-fucoidan is mainly composed of sulphated esters of fucose, while U-fucoidan comprises about 20 % of glucuronic acid.

The pharmaceutical composition or medicament according to the present invention adjusted for parenteral topical use may be provided as a liquid preparation selected from the group consisting of a lotion, an aqueous solution including a solution for inhalation, a spray or sprayable solution, liquid drops, and a gargle solution, or as a foam or as a semi-solid preparation selected from the group consisting of a cream, an ointment, and a gel.

The pharmaceutical composition or medicament according to the present invention may, however, also be adjusted for oral administration, e.g. prepared in the form of semi-solid or solid dosage units such as tablets, capsules, dragées, lozenges, chewing gums, or any other orally ingestible galenic form, wherein the antiviral effect of the preparation is intended to occur primarily in the oral cavity, particularly in the saliva and the mucosal tissues of the mouth.

Where the composition is for topical use and is a liquid a foam, or a semi-solid preparation it typically comprises in its ready-for-use form the sulphated polysaccharide antiviral active ingredient in an amount of from 0.01 to 5% , i.e. from 0.1 mg/ml to 50 mg/ml, most preferably from 0.01 to 1% (0.1 - 10 mg/ml) by weight, and typically from 0.01 to 0.5% (0.1 - 5 mg/ml) relative to the total volume of the composition. While in case of carrageenans as antiviral active ingredient the upper threshold value for the carrageenan concentration is mainly determined by the increasing viscosity of the preparation caused by the carrageenan component, it is decisive for the antiviral efficacy of the preparation that the concentratation of the sulphated polysaccharides at the site of action, e.g. at the skin or at a mucosal tissue or in the oral saliva, upon topical administration is at least 10 ug/ml, or at least 100 ug/ml, or at least 400 ug/ml. Therefore, taking into account that dilution effects by the moisture of the skin, the mucosal tissues, or the saliva will occur the ready-for-use preparations need to comprise the active antiviral ingredient at a concentration that is slightly or substantially higher than the corresponding therapeutically active IC90 concentration determined in *in vitro* assays.

Where the composition is prepared as solid dosage unit it typically comprises in its ready-for-use form carrageenan in an amount of from 0.01 to 10%, preferably from 0.01 to 5%, most preferably from 0.1 to 2% by weight, relative to the total weight of the composition. Given that the weight of a dosage unit of a solid preparation (e.g. a tablet, capsule, dragée, lozenge, chewing gum) may vary within a broad range, i.e. typically within a range of from 0.1 to 5 g per dosage unit, it is particularly preferred to provide the antiviral active ingredient in an amount of from 1 mg to 100mg per dosage unit, or from 2 to 50 mg, or from 5 to 20 mg per dosage unit. Such preparations will ensure that the effective concentration of the antiviral active ingredient at the site of action, e.g. in the saliva and the mucosal tissues in the mouth, will reach and typically substantially exceed the IC90 values, i.e. the concentration values required for a 90% inhibition of viral infection of experimental eukaryotic cells in an *in vitro* assay, as well as the experimentally determined minimal antiviral effective concentrations of 10 ug/ml, 100 ug/ml or 400 ug/ml.

While the pharmaceutical composition or medicament prepared in accordance with the present invention comprises the sulphated polysaccharide component in an antiviral effective amount it is preferred that the sulphated polysaccharide component is the sole antiviral active ingredient of the composition or medicament.

For some applications the composition is substantially comprised solely of iota-carrageenan, or solely of kappa-carrageenan, or solely of lambda-carrageenan, while for other applications the composition may comprise mixtures thereof. In particular, in one embodiment the composition may comprise more than 50%, preferably more than 70%, and especially up to 95% (w/w) by dry weight of iota-carrageenan, relative to the total dry weight of all carrageenans present in the composition, the remaining carrageenans being of the kappa- and/or lambda-type. In another embodiment the composition may comprise more than 50%, preferably more than 70%, and especially up to 95% (w/w) by dry weight of kappa-carrageenan, relative to the total dry weight of all carrageenans present in the composition, the remaining carrageenans being of the iota- and/or lambda-type. In yet another embodiment, the composition may comprise more than 50%, preferably more than 70%, and especially up to 95% (w/w) by dry weight of lambda-carrageenan, relative to the total dry weight of all carrageenans present in the composition, the remaining carrageenans being of the kappa- and/or iota-type.

The antiviral carrageenan compositions in accordance with the present invention may further comprise at least one pharmaceutically acceptable carrier and/or additive suitable and permitted for medical application.

The carrier may be a diluent, e.g. water, saline, optionally phosphate buffered saline (PBS), an excipient, or another vehicle which facilitates the administration of the composition. Where the composition is solid, semi- solid or fluid the groups of carriers and additives, respectively, may comprise but are not limited to SiO₂, TiO₂, a binder such as microcrystalline cellulose, polyvinylpyrrolidone, gum tragacanth, gelatine, starch, lactose, lactose monohydrate, alginic acid or maize starch; a lubricant or surfactant like magnesium stearate or sodium lauryl sulphate; a glidant like e.g. colloidal silicon dioxide; a sweetening agent such as sucrose or saccharin.

Still further additives may be selected from the group consisting of physiologically acceptable preservatives, pharmaceutically acceptable alkali metal salts like sodium, potassium, lithium or ammonium chlorides, buffers or pH adjusting agents such as citric acid, acetic acid, fumaric acid, hydrochloric acid, malic acid, nitric acid, phosphoric acid, propionic acid, sulphuric acid and tartaric acid, and combinations of said acids.

Typically, where pharmaceutically acceptable salts are present in the composition, they are provided in a concentration of not more than 1.5 %, preferably not more than 1%, and usually of not more than 0.6 % (w/v). Salt concentrations beyond 1.5% would impair at least the carrageenan's antiviral efficacy.

In the compositions of the present invention the carrageenan itself may be present as a salt, too, preferably as a sodium salt.

It is preferred that the present compositions for prophylactic or therapeutic medical application be provided as sterile, germ-free, pathogen-free, pyrogenfree and allergen-free preparations.

Carrageenan was found to be non-toxic upon oral or dermal administration, or upon inhalation, even when applied at extremely high doses. It was therefore classified as "generally recognized as safe" (GRAS) by the Food and Drug Administration (FDA).

It is also within the scope of the present invention to use a sulphated polysaccharide component comprising iota-, kappa-, or lambda-carrageenan, or cellulose sulphate, or dextran sulphate, or any mixture of said sulphated polysaccharides, as an antiviral agent in the manufacture of pharmaceutical compositions or medicaments, wherein the sulphated polysaccharide component is present in an antiviral effective amount in combination with at least one specific drug usually applied in the prophylaxis or therapy of infectious and/or inflammatory diseases, allergies and/or conditions of an impaired or suppressed immune system.

It is preferred, however, that the sulphated polysaccharide component, especially the carrageenan component comprising iota-, kappa-, or lambda-carrageenan, or any mixture of the foregoing carrageenans, is the sole or predominant active antiviral ingredient in compositions for the prophylactic or therapeutic intervention of coronavirus infections, including SARS-CoV-2 infections, according to the present invention.

Parenteral administration of the pharmaceutical composition according to the present invention is preferred, particularly administration in the form of a nasal spray, a mouth spray, a liquid solution for inhalation, a foam, liquid drops or gargle solutions. Nasal and mouth sprays as well as foams and gargle solutions are particularly useful in the prophylaxis of coronavirus infection, whereas preparations for inhalation as well as gargle solutions, tablets, capsules, dragées, lozenges, and chewing gums are also useful in the therapeutic treatment of infected individuals.

Inhalation of preparations manufactured in accordance with the present invention may substantially alleviate the severe respiratory symptoms caused by SARS-CoV-2 observed with many COVID-19 patients and especially with high-risk patients, i.e. patients selected from the group of COPD-patients, asthma patients, individuals suffering from allergies, or from impaired immune systems, from impaired cardiac systems, or from impaired pulmonary systems, and transplantation patients.

In addition, the present pharmaceutical compositions may be applied, e.g. sprayed - much like a sanitizer or disinfectant - onto solid surfaces including hands, fingers, gloves, hygiene tissues or papers including nasal tissues or papers, in order to exert a virucide effect at least to some extent, thus contributing to reducing an individual's repeated self-infection by contaminated fingertips and also to reduce viral distribution among different individuals that are in close, e.g. hand-to-hand, contact with each other.

Further items coated, impregnated or soaked with a pharmaceutical composition in accordance with the present invention comprise cotton swabs, dust masks or sanitary or medical facial masks. Even lipsticks can be formulated to contain an antiviral effective amount of the present pharmaceutical composition comprising one or more of the sulphated polysaccharide compounds in accordance with the invention. These hygiene or sanitation articles can be used prophylactically or for therapeutical treatment against a coronavirus infection and may assist in the prevention or reduction of a risk of infection.

It is known in the art that attachment and entry of SARS-CoV-2 is mediated by the spike glycoprotein (SGP). In addition to its interaction with its receptor, i.e. human angiotensin converting enzyme 2 (ACE2), the spike glycoprotein has been found to also bind to glycosaminoglycans like heparan sulphate, which is found on the surface of virtually all mammalian cells.

In order to further explain the present invention the following examples are set forth hereinafter. They confirm *inter alia* a strong inhibitory effect of the carrageenan polymers on the infection of ACE2-receptor bearing mammalian cells in a SARS-CoV-2 Spike Pseudotyped Lentivirus assay.

It may be inferred from the strong infection inhibitory effect of the sulphated polysaccharide polymers that the intervention of the polymers with the SGP-driven entry of the pseudotyped lentivirus into the mammalian target cells is mediated by both the negative charges of the sulphated galactose-based polymers and by certain structural relationships between the polymers on one hand and the ACE2 receptor of the target cell and/or the spike glycoprotein of the virus on the other hand.

### EXAMPLE 1: Inhibitory Effect of Iota-Carrageenan on SARS-CoV2 Infection

The inhibitory effect of iota-carrageenan on SARS-CoV2 infection has been determined in an in vitro neutralization assay based on the commercially available SARS-CoV-2 spike pseudotyped lentivirus assay (BPS Bioscience, Inc., Catalog # 79942; URL: https://bpsbioscience.com/spike-sars-cov-2-pseudotyped-lentivirus-luc-reporter-79942). Using the modified lentivirus system allows for measuring the transduction-inhibitory activity of antiviral agents against SARS-CoV-2 in a biosafety level 2 facility.

According to the manufacturer's data sheet the SARS-CoV-2 Spike Pseudotyped Lentivirus comprises a SARS-CoV-2 Spike (Genbank Accession #QHD43416.1) as the envelope glycoproteins. In addition, these pseudovirions also contain the firefly luciferase gene driven by a CMV promoter. Therefore, the spike-mediated cell entry (= transduction equivalent to infection) can be measured via luciferase reporter activity.

Experimental design:
Incubation media were used according to the assay manufacturer's recommendations, i.e. HEK293 growth medium MEM with 10% FBS, 0.1 mM nonessential amino acids, 1 mM sodium pyruvate. Commercially available Gelcarin^{®} PH379 has been used as a iota-carrageenan source. Its composition of matter has been determined by 1H-NMR spectroscopy to comprise 96.9 mol% of iota+ nu-type subunits, and 3.1 mol% of kappa-type subunits. In a 96-well-plate approximately 7500 ACE2-HEK293 cells/well were infected with 5ul per well of a mixture of SARS-CoV-2 Spike pseudotyped lentivirus (luciferase reporter) and iota-carrageenan polymer or carboxymethyl cellulose (CMC) polymer at varying polymer concentrations. Virus was incubated either solely with buffer (controls) or with buffer comprising different concentrations of iota-carrageenan or carboxymethyl cellulose (CMC) for 30 minutes prior to infection, i.e. 400, 133, 40, 13, 4, and 1.3 ug/ml iota-carrageenan, and 400, 40 and 4 ug/m of CMC. 24 hours after infection, the medium was changed to fresh HEK growth medium. 48 hours after infection the cells were lysed by freeze/thaw cycles before luciferase reagent was added to the wells to measure the luciferase activity. The infection rate or infection efficiency determined by measuring the luciferase activity indicates the percentage of infected cells in the presence of the antiviral protective carrageenan component, and thus the carrageenan component's protective efficacy against SARS-CoV-2 infection.

The results are depicted in Fig. 1, wherein maximum luciferase activity determined from cells infected with the virus in the absence of the protective carrageenan component (mock treatment) was set as 100% (= positive control), whereas the luciferase activity determined from uninfected cells was set as 0% (= negative control). Inhibition of infection by iota-carrageenan (black bars) was compared with the corresponding activity of carboxymethyl cellulose (CMC, hatched bars). Luciferase data were corrected for metabolic activity determined with Alamar blue using a parallel plate with identical set-up. Different polymer concentrations are indicated at the x-axis, and the corrected luciferase activity values indicating the percentage of infected ACE2-HEK293 cells are displayed on the y-axis. The data represent means of duplicates.

It can be taken from Fig. 1 that CMC had no infection inhibitory effect at 400 and 40 ug/ml, and a weak inhibitory effect at only 4 ug/ml. Whereas iota-carrageenan was highly effective at the highest concentration of 400 ug/ml resulting in a reduction of infection of more than 90%, relative to the unprotected positive control, while at concentrations of 133, 40 and 13 ug/ml iota-carrageenan still produced a sound inhibitory effect of approximately 80%, relative to the unprotected positive control. Only at the lowest concentrations of 4 and 1.3 ug/ml the infection inhibitory efficacy of iota-carrageenan seemed to fade away to some extent, even though at these concentrations iota-carrageenan was still able to keep the number of infected cells around 30% below the unprotected positive control.

It appears therefore that iota-carrageenan qualifies as a useful agent in the manufacture of a pharmaceutical composition or medicament for the prophylactic or therapeutic treatment of mammals, in particular human individuals, at risk of or suffering from coronavirus infection, and especially from infection with SARS CoV2.

### EXAMPLE 2: Protective Efficacy of Iota-Carrageenan Against SARS-CoV-2 at Varying Concentrations

The experimental setup of Example 1 was repeated with the following modifications:
7000 HEK293 cells expressing the ACE-2 receptor were infected with 750 infectious particles of the SARS-CoV-2 pseudotyped lentivirus expressing luciferase. Virus was incubated either solely with buffer (controls) or with buffer comprising different amounts of iota-carrageenan or hydroxypropylmethyl cellulose HPMC for 30 minutes prior to infection, i.e. 100, 33, 10, 3.3, 1 und 0.33 ug/ml of iota-carrageenan, and 100 ug/ml of HPMC (Fig. 2, x-axis). 24 h after infection the medium was replaced with fresh growth medium. 48 h after infection the cells were lysed by freeze/thaw-cycles, luciferase reagent was added and luciferase enzyme activity was determined and evaluated against the positive and negative controls.

In Fig. 2 the resulting values of infection rate (y-axis) were corrected for metabolic activity determined by Alamar blue on an identical plate. Cells mock-infected were set to 0%, while mock-treated infected cells were set to 100%. The bars represent means of triplicates with the standard deviation indicated.

It can be taken from Fig. 2 that iota-carrageenan is highly protective in a concentration range *in situ* of from 10 ug/ml to 100 ug/ml, whereas at very low concentrations of 1 ug/ml or less the inhibitory/protective efficacy against SARS-CoV-2 infection seems to fade away. It appears that there is a sound dose-dependent efficacy of iota-carrageenan.

HPMC applied at a single concentration of 100 ug/ml exerts a weak infection-inhibitory effect only.

### EXAMPLE 3: Comparison of Protective Efficacy of Various Negatively Charged Polymers Against Infection With SARS-CoV-2

The experimental setup of Example 2 was repeated with the following modifications:

In addition to iota-carrageenan also kappa-carrageenan, purified lambda-carrageenan, high molecular-weight (HMW) fucoidan, cellulose sulphate, heparan sulphate, and dextran sulphate were enrolled to the experimental setup at 3 different concentrations each, i.e. at 100 ug/ml, 10 ug/ml and 1 ug/ml. Kappa-carrageenan was obtained from commercially available Gelcarin^{®} PH91 1 (1H-NMR determination of subunits: 83.6 mol% kappa-type, 16.4 mol% nu + iota-type); lambda-carrageenan was obtained from commercially available Viscarin^{®} PH109 (1H-NMR determination of subunits: 41.8 mol% kappa-type + 0.7 mol% mu-type, 27.3 mol% iota-type + 10.6 mol% nu-type, 19.6 mol% lambda-type). For the purpose of the present experimental use the lambda-carrageenan material (Viscarin^{®} PH109) has been further purified to comprise at least about 30%, typically about 45 - 55 mol% of lambda-type subunits, relative to the total of carrageenan type subunits in the experimental lambda-carrageenan material.

The results are depicted in Fig. 3.

Also, in order to better understand the foregoing protective activities of the polymers further polymeric agents have been tested under the same experimental conditions, i.e. carboxymethyl cellulose (CMC), hydroxypropylmethyl cellulose (HPMC), and galactose-4-sulphate (Gal-4-SO4), each of these polymers at a concentration of 100 ug/ml.

Surprisingly, while the carrageenans as well as cellulose sulphate and dextran sulphate exert highly protective efficacy at least at the highest concentrations, and particularly within a range of from 10 ug/ml to 100 ug/ml, fucoidan seems to be only poorly protective and heparan sulphate does not exert any protective effect at all.

Similarly, the comparative polymers CMC, HPMC, and Gal-4-SO4 are only poorly effective in the protection of the mammalian target cells against infection with modified lentivirus mimicking SARS-CoV-2.

The best results are achieved with iota- and purified lambda-carrageenan, and with cellulose sulphate and dextran sulphate. It shall be noted at this occasion that in previous studies on the antiviral efficacy of lambda-carrageenan, this polymer has been found to exert only poor antiviral efficacy, if at all. Most recent findings of the present inventors indicate that this lack of antiviral activity of lambda-carrageenan may have been due to the quality of commercially available lambda-carrageenan, which indeed contained only very low amounts, i.e. only up to about 5 - 10% by weight, of lambda-carrageenan. Most of the material sold as "lambda carrageenan" was other polymers. Therefore, the lambda-carrageenan material used in the Examples has been specifically purified to contain at least 30 mol%, preferably about 45 - 55 mol%, of lambda-type subunits, relative to the total of carrageenan-type disaccharide repeating subunits present in the experimental lambda-carrageenan starting material.

As a side note, it may be mentioned that enzymatic treatment of the Viscarin^{®} PH109 lambda-carrageenan material with kappa-carrageenase and subsequent 1H-NMR spectroscopic determination of the distribution of carrageenan-typ disaccharide subunits seems to confirm that the majority of lambda-type subunits is linked with kappa-type subunits to form lambda/kappa or kappa/lambda-polymeric chains, and only to a minor extent with iota-type subunits to form lambda/iota or iota/lambda or kappa/iota/lambda or lambda/kappa/iota type polymeric chains.

### EXAMPLE 4: Antiviral efficacy of lozenges comprising iota-carrageenan

In a monocentric, open label, prospective clinical trial, 31 healthy subjects were included to suck an iota-carrageenan containing lozenge. Saliva samples from 27 subjects were used for ex vivo efficacy analysis. The primary objective was to assess whether the mean iota-carrageenan concentration of the saliva samples exceeded 5 *µ*g/ml, which is the concentration known to reduce replication of human rhinovirus (hRV) subtypes 1a and 8 by 90 % (IC90). The iota-carrageenan concentration of the saliva samples was analyzed by UV-Vis spectroscopy. The antiviral effectiveness of the individual saliva samples was determined in vitro against a panel of respiratory viruses including hRV1a, hRV8, human Coronavirus OC43, influenza virus A H1N109pdm, coxsackievirus A10, parainfluenza virus 3, and SARS-CoV-2, using standard virological assays.

27 eligible male and non-pregnant female subjects with no signs of an acute respiratory infection were included in the study. 2/3 of the probands were female and 1/3 were male. The median age was 52.5 (± 13.7) years, the mean body mass index was 27.06 (± 5.9) kg/m2.

Subjects had to collect native saliva before sucking one lozenge with an iota-carrageenan content of 10 mg. During sucking of the lozenge subjects donated their saliva instead of swallowing it. When the collected saliva volume reached about 5 ml sucking was terminated and samples were stored at -20°C.

Native saliva samples from the participants were pooled whereas carrageenan-treated saliva samples were analyzed separately. All samples were diluted 1:2 with distilled water, heat-sterilized and aliquoted. One aliquot was used for determination of carrageenan concentration, and the remaining aliquots were used in the antiviral activity assays.

The iota-carrageenan concentration of the heat-inactivated samples was determined by means of UV/VIS detection after complex formation with methylene blue (MB). Briefly, methylene blue interacts with iota-carrageenan at low concentrations to form a water-soluble meta-chromic complex. The complexation results in a change of the dye from blue (absorptions maxima at 610 and 666 nm) to purple with a maximum absorption wavelength of 559 nm. The absorbance values at these wavelengths are proportional to the iota-carrageenan concentration. Also, pooled native samples were tested accordingly. The measured iota-carrageenan concentrations of the verum samples were corrected with the signal of the native samples (baseline corrected).

Antiviral activity / replication inhibition assay - SARS-CoV-2:
For determining the antiviral activity, Vero B4 cells were maintained in Dulbecco's Modified Eagle's Medium containing 10 % (v/v) inactivated fetal calf serum, 2 mM I-glutamine, 100 U/ml penicillin, and 100 *µ*g/ml streptomycin. Per 24 well plate, 4.8 × 10⁶ Vero B4 cells were resuspended in 3.6 ml medium and infected in FCS-free DMEM with an MOI of 0.02 of the field isolate SARS-CoV-2PR-1 for 1 h at 37 °C under gentle rotation. Afterwards, the inoculum was removed. After washing with PBS, the cells were resuspended in the amount of medium necessary to seed 2 × 10⁵ cells/well. Infected cells were seeded in 24 well plates and incubated with carrageenan-treated saliva at 15, 10, 5 and 2.5 *µ*g/ml iota-carrgeenan. At 72 h post-infection, supernatants were harvested, incubated for 10 minutes at 95 °C and analyzed via q-RT-PCR.

Iota-carrageenan was obtained from Dupont (formerly FMC Biopolymers, Philadelphia, PA). The dry polymer powders were dissolved in cell culture water to a final iota-carrageenan concentration of 2.4 mg/ml containing 0.5 % NaCl. This stock solution was sterile filtered through a 0.22mm filter and stored at 4°C until use. Iota-carrageenan was used as a reference in all assays. The quality of iota-carrageenan was analyzed via 1H-NMR, which confirmed a content of 92.5 mol% of iota-type disaccharide repeating subunits and minor quantities of kappa- and nu-type subunits.

The mean iota-carrageenan concentration of the clinical saliva samples was determined as 667.8 *µ*g/ml which exceeded the IC90/MIC concentrations of all tested viruses by a factor of from 60 to about 30,000-fold. This indicates that by sucking an iota-carrageenan containing lozenge much more iota-carrageenan is released into the saliva than necessary to neutralize or inactivate the most common respiratory viruses, and even SARS-CoV-2.

More specifically, the mean iota-concentration present in the saliva samples exceeded the concentration needed to inhibit 90% of viral replication of SARS-CoV-2 by 120.5-fold (p < 0.001). Replication competent SARS-CoV-2 was effectively neutralized when incubated with iota-carrageenan treated saliva.

These data support and endorse the results disclosed in Examples 1 to 3 for SARS-CoV-2 spike pseudo typed lentivirus, which confirmed that iota-carrageenan inhibits cell entry of the SARS-CoV-2 spike pseudo typed lentivirus and replication of SARS-CoV-2 with an IC50 of < 2.6 *µ*g/ml iota-carrageenan.

It has further been found that the mean retention time of carrageenans in the nasal or oral mucosa is within a range of from 1 to 6 hours, and typically within a range of from 3 to 4 hours, whereas complete neutralization of the viruses by the sulphated polysaccharides of the present invention usually occured within 20 minutes.

**Table 1: Antiviral effectiveness of the saliva samples against various viruses. IC90 and MIC in (µg/ml).**

| | HRV1a (IC90) | HRV8 (IC90) | hCoV OC43 (MIC) | IV A H1N1n (MIC) | Coxsackie virus A10 (IC90) | PIV3 (MIC) | SARS-CoV-2 (IC90) |
|---|---|---|---|---|---|---|---|
| Mean IC90/MIC | 9.5 | 2.38 | 0.025 | 0.66 | 14.79 | 0.07 | 7.46 |
| SD | 3.71 | 3.00 | 0.012 | 0.25 | 11.02 | 0.01 | 4.72 |
| Minimum | 4.76 | 0.69 | 0.007 | 0.24 | 5.97 | 0.02 | 1.47 |
| Lower quartile | 6.79 | 1.17 | 0.024 | 0.52 | 8.13 | 0.07 | 4.75 |
| Median | 8.98 | 1.36 | 0.024 | 0.80 | 8.74 | 0.07 | 5.49 |
| Upper quartile | 11.22 | 2.44 | 0.024 | 0.80 | 21.32 | 0.07 | 9.89 |
| Maximum | 18.9 | 16.57 | 0.081 | 0.80 | 49.19 | 0.07 | 22.56 |

| | | | | | | | |
|---|---|---|---|---|---|---|---|
| Notes: The respective viruses were incubated with a dilution series of the treated saliva samples before infection of a susceptible cell line. (N = 27). Abbreviations: IV, influenza virus; MIC, minimal inhibitory concentration; IC90, 90 % maximal inhibitory concentration; SD, standard deviation; N, population size. | | | | | | | |

**Table 2: Mean difference / excess factors of iota-carrageenan concentration in treated saliva relative to IC90/MIC (95 % CI)**

| Virus tested | Mean Difference (*µ*g/ml) | Mean x-fold excess | p-value |
|---|---|---|---|
| HRV1A | 658.20 (571.7; 744.7) | 81.8 (64.9; 98.6) | < 0.001 |
| HRV8 | 665.42 (578.68 752.16) | 463.2 (353.8; 572.6 | < 0.001 |
| hCoV OC43 | 667.77 (581.49 754.06) | 30350.9 (22909.8; 37792.0) | < 0.001 |
| IV A H1N1n | 667.14 (580.87 753.42) | 1323.6 (964.5; 1682.7) | < 0.001 |
| Coxsackie A10 | 653.01 (568.07 737.95) | 60.0 (47.4; 72.6) | < 0.001 |
| PIV3 | 667.7 (581.4; 754.0) | 10154.0 (8563.0; 11746.0) | < 0.001 |
| SARS-CoV-2 | 660.3 (573.9; 746.8) | 120.5 (93.2; 147.8) | < 0.001 |

| | | | |
|---|---|---|---|
| Notes: Carrageenan concentrations of the saliva samples were compared to concentrations needed to inhibit at least 90 % of virus binding/replication (IC90, MIC). p-values from paired t-tests indicate statistical significance of the differences. Abbreviations: Cl, confidence interval; CIL, lower confidence interval; CIU, upper confidence interval; IV, influenza virus. | | | |

Conclusions:
Sucking an iota-carrageenan containing lozenge releases sufficient iota-carrageenan to neutralize and inactivate the most abundant respiratory viruses as well as pandemic SARS-CoV-2. The lozenges are therefore an appropriate measure to reduce the viral load at the site of infection.

## Claims

1. A sulphated polysaccharide selected from the group consisting of iota-carrageenan, kappa-carrageenan, lambda-carrageenan, cellulose sulphate, and dextran sulphate, for use as an antiviral active ingredient in a pharmaceutical composition or medicament for the prophylactic or therapeutic application against a SARS-CoV-2 infection in a mammal, especially in a human individual, wherein
a) said composition is in the form of a liquid, a foam, or a semi-solid preparation, and the antiviral active ingredient is present therein in a concentration of at least 10 ug/ml, at least 100 ug/ml, or at least 400 ug/ml;
or else, wherein
b) said composition is in the form of a solid dosage unit, and the antiviral active ingredient is present therein in an amount of at least 0.5 mg, at least 2 mg, at least 5 mg, at least 10 mg, or at least 20 mg per dosage unit.

2. The sulphated polysaccharide for use according to claim 1, wherein the antiviral active ingredient comprises at least one, optionally a mixture of at least two of said sulphated polysaccharides.

3. The sulphated polysaccharide for use according to claim 1 or 2, wherein
- said liquid or semi-solid composition is selected from the group consisting of a skin lotion, an aqueous solution for disinfection, an aqueous solution for inhalation, a spray or sprayable solution, liquid drops, and a gargle solution;
- said semi-solid composition is selected from the group consisting of a gel, a cream, and an ointment; and
- said solid dosage unit is selected from the group consisting of a tablet, a lozenge, a dragée, a capsule, and a chewing gum.

4. A pharmaceutical composition or medicament comprising at least one sulphated polysaccharide selected from the group consisting of iota-carrageenan, kappa-carrageenan, lambda-carrageenan, cellulose sulphate, and dextran sulphate, as an antiviral active ingredient for use in the prophylactic or therapeutic application against a SARS-CoV-2 infection in a human individual or non-human animal, wherein
a) said composition or medicament is in the form of a liquid, a foam or a semi-solid preparation and the antiviral active ingredient is present therein in a concentration of at least 10 ug/ml, at least 100 ug/ml, or at least 400 ug/ml;
or else, wherein
b) said composition or medicament is in the form of a solid dosage unit and the sulphated polymer is present therein in an antiviral effective amount of at least 0.5 mg, at least 2 mg, at least 5 mg, at least 10 mg, or at least 20 mg, per dosage unit.

5. The pharmaceutical composition or medicament for use according to claim 4,
wherein
said liquid or semi-solid composition is selected from the group consisting of a skin lotion, an aqueous solution for disinfection, an aqueous solution for inhalation, a spray or sprayable solution, liquid drops, and a gargle solution;
wherein said semi-solid composition is selected from the group consisting of a gel, a cream, and an ointment; and
wherein said solid dosage unit is selected from the group consisting of a tablet, a lozenge, a dragée, a capsule, and a chewing gum.

6. The pharmaceutical composition or medicament for use according to claim 4 or 5, which comprises a carrageenan component in an antiviral effective amount, wherein said carrageenan component is either iota-, kappa- or lambda-carrageenan, or a mixture of at least two of iota-, kappa- and lambda-.

7. The pharmaceutical composition or medicament for use according to any one of claims 4 to 6, wherein the lambda-carrageenan is purified to comprise at least 30 mol%, preferably 45 to 55 mol% of lambda-type repeating disaccharide subunits, relative to the total of iota-, kappa-, lambda-, mu- and nu-type repeating disaccharide subunits present in the lambda-carrageenan material.

8. The pharmaceutical composition or medicament for use according to any one of claims 4 to 7, wherein said composition or medicament comprises at least one pharmaceutically acceptable carrier and/or additive, said additive preferably selected from sodium and potassium chloride and being present in the composition in an amount of 1.5 %wt or less, or of 1.0 %wt or less, or of 0.6 % or less.

9. The pharmaceutical composition or medicament for use according to any one of claims 4 - 8, wherein the composition in a liquid form is attached by either coating or impregnation to a solid surface of a hygiene or sanitary item.

10. The pharmaceutical composition or medicament for use according to claim 9, wherein said hyiene or sanitary item is selected from the group consisting of a hygiene or sanitary glove, a hygiene or sanitary tissue or paper, a cotton swab, a dust mask, a sanitary facial mask, and a medical facial mask.

11. The pharmaceutical composition or medicament for use according to claim 4, wherein said human individual is a high-risk patient selected from the group consisting of a COPD-patient, an asthma patient, a person with allergies, a person with impaired immune, cardiac, or pulmonary system, and a transplantation patient, or wherein said human individual is a member of healthcare personell in hospitals, pharmacies and doctor's offices, or a person who occupationally gets into close contact with infected or potentially infected human individuals or non-human animals.

## Patentansprüche

1. Sulfatiertes Polysaccharid, ausgewählt aus der Gruppe bestehend aus lota-Carrageenan, Kappa-Carrageenan, Lambda-Carrageenan, Cellulosesulfat und Dextransulfat, zur Verwendung als antiviraler Wirkstoff in einer pharmazeutischen Zusammensetzung oder einem Medikament zur prophylaktischen oder therapeutischen Anwendung gegen eine SARS-CoV-2-Infektion bei einem Säugetier, insbesondere bei einem menschlichen Individuum, wobei
a) die Zusammensetzung in Form einer Flüssigkeit, eines Schaums oder einer halbfesten Zubereitung vorliegt, und der antivirale Wirkstoff darin in einer Konzentration von mindestens 10 ug/ml, mindestens 100 ug/ml oder mindestens 400 ug/ml vorhanden ist;
oder sonst, wobei
b) die Zusammensetzung in Form einer festen Dosierungseinheit vorliegt und der antivirale Wirkstoff darin in einer Menge von mindestens 0,5 mg, mindestens 2 mg, mindestens 5 mg, mindestens 10 mg oder mindestens 20 mg pro Dosierungseinheit vorhanden ist.

2. Sulfatiertes Polysaccharid zur Verwendung nach Anspruch 1, wobei der antivirale Wirkstoff mindestens ein, gegebenenfalls ein Gemisch aus mindestens zwei der sulfatierten Polysaccharide umfasst.

3. Sulfatiertes Polysaccharid zur Verwendung nach Anspruch 1 oder 2, wobei
- die flüssige oder halbfeste Zusammensetzung ausgewählt ist aus der Gruppe bestehend aus einer Hautlotion, einer wässrigen Lösung zur Desinfektion, einer wässrigen Lösung zur Inhalation, einem Spray oder einer sprühbaren Lösung, flüssigen Tropfen und einer Gurgellösung;
- die halbfeste Zusammensetzung ausgewählt ist aus der Gruppe, die aus einem Gel, einer Creme und einer Salbe besteht; und
- die feste Dosierungseinheit ausgewählt ist aus der Gruppe bestehend aus einer Tablette, einem Lutschbonbon, einem Dragee, einer Kapsel und einem Kaugummi.

4. Pharmazeutische Zusammensetzung oder Arzneimittel, umfassend mindestens ein sulfatiertes Polysaccharid, ausgewählt aus der Gruppe bestehend aus lota-Carrageenan, Kappa-Carrageenan, Lambda-Carrageenan, Cellulosesulfat und Dextransulfat, als antiviralen Wirkstoff zur Verwendung bei der prophylaktischen oder therapeutischen Anwendung gegen eine SARS-CoV-2-Infektion bei einem menschlichen Individuum oder einem nicht-menschlichen Tier, wobei
a) die Zusammensetzung oder das Medikament in Form einer Flüssigkeit, eines Schaums oder einer halbfesten Zubereitung vorliegt und der antivirale Wirkstoff darin in einer Konzentration von mindestens 10 ug/ml, mindestens 100 ug/ml oder mindestens 400 ug/ml vorhanden ist;
oder sonst, wobei
b) die Zusammensetzung oder das Medikament in Form einer festen Dosierungseinheit vorliegt und das sulfatierte Polymer darin in einer antiviral wirksamen Menge von mindestens 0,5 mg, mindestens 2 mg, mindestens 5 mg, mindestens 10 mg oder mindestens 20 mg pro Dosierungseinheit vorhanden ist.

5. Pharmazeutische Zusammensetzung oder Arzneimittel zur Verwendung nach Anspruch 4, wobei
die flüssige oder halbfeste Zusammensetzung ausgewählt ist aus der Gruppe bestehend aus einer Hautlotion, einer wässrigen Lösung zur Desinfektion, einer wässrigen Lösung zur Inhalation, einem Spray oder einer sprühbaren Lösung, flüssigen Tropfen und einer Gurgellösung;
wobei die halbfeste Zusammensetzung aus der Gruppe ausgewählt ist, die aus einem Gel, einer Creme und einer Salbe besteht; und
wobei die feste Dosierungseinheit aus der Gruppe ausgewählt ist, die aus einer Tablette, einer Lutschtablette, einem Dragee, einer Kapsel und einem Kaugummi besteht.

6. Pharmazeutische Zusammensetzung oder Arzneimittel zur Verwendung nach Anspruch 4 oder 5, die/der eine Carrageenan-Komponente in einer antiviral wirksamen Menge umfasst, wobei die Carrageenan-Komponente entweder lota-, Kappa- oder Lambda-Carrageenan oder eine Mischung aus mindestens zwei von lota-, Kappa- und Lambda-Carrageenan ist.

7. Pharmazeutische Zusammensetzung oder Medikament zur Verwendung nach einem der Ansprüche 4 bis 6, wobei das lambda-Carrageenan so gereinigt ist, dass es mindestens 30 Mol-%, vorzugsweise 45 bis 55 Mol-%, an sich wiederholenden Disaccharid-Untereinheiten vom lambda-Typ enthält, bezogen auf die Gesamtheit der im lambda-Carrageenan-Material vorhandenen sich wiederholenden Disaccharid-Untereinheiten vom iota-, kappa-, lambda-, mu- und nu-Typ.

8. Pharmazeutische Zusammensetzung oder Medikament zur Verwendung nach einem der Ansprüche 4 bis 7, wobei die Zusammensetzung oder das Medikament mindestens einen pharmazeutisch annehmbaren Träger und/oder Zusatzstoff umfasst, wobei der Zusatzstoff vorzugsweise aus Natrium- und Kaliumchlorid ausgewählt ist und in der Zusammensetzung in einer Menge von 1,5 Gew.-% oder weniger, oder von 1,0 Gew.-% oder weniger, oder von 0,6 % oder weniger vorhanden ist.

9. Pharmazeutische Zusammensetzung oder Medikament zur Verwendung nach einem der Ansprüche 4 bis 8, wobei die Zusammensetzung in flüssiger Form entweder durch Beschichtung oder Imprägnierung an einer festen Oberfläche eines Hygiene- oder Sanitärartikels angebracht ist.

10. Pharmazeutische Zusammensetzung oder Medikament zur Verwendung nach Anspruch 9, wobei der Hygiene- oder Sanitärgegenstand aus der Gruppe ausgewählt ist, die aus einem Hygiene- oder Sanitärhandschuh, einem Hygiene- oder Sanitärtuch oder -papier, einem Wattestäbchen, einer Staubmaske, einer sanitären Gesichtsmaske und einer medizinischen Gesichtsmaske besteht.

11. Pharmazeutische Zusammensetzung oder Arzneimittel zur Verwendung nach Anspruch 4, wobei das menschliche Individuum ein Hochrisikopatient ist, ausgewählt aus der Gruppe bestehend aus einem COPD-Patienten, einem Asthmapatienten, einer Person mit Allergien, einer Person mit beeinträchtigtem Immun-, Herz- oder Lungensystem und einem Transplantationspatienten, oder wobei das menschliche Individuum ein Mitglied des Gesundheitspersonals in Krankenhäusern, Apotheken und Arztpraxen ist, oder eine Person, die beruflich in engen Kontakt mit infizierten oder potentiell infizierten menschlichen Individuen oder nicht-menschlichen Tieren kommt.

## Revendications

1. Polysaccharide sulfaté choisi dans le groupe constitué de iota-carraghénane, kappa-carraghénane, lambda-carraghénane, sulfate de cellulose et sulfate de dextrane, destiné à être utilisé comme principe actif antiviral dans une composition pharmaceutique ou un médicament pour l'application prophylactique ou thérapeutique contre une infection par le SRAS-CoV-2 chez un mammifère, en particulier chez un individu humain, dans lequel
a) ladite composition se présente sous la forme d'un liquide, d'une mousse ou d'une préparation semi-solide, et le principe actif antiviral y est présent à une concentration d'au moins 10 ug/ml, d'au moins 100 ug/ml, ou d'au moins 400 ug/ml;
ou bien, dans laquelle
b) ladite composition se présente sous la forme d'une unité de dosage solide, et le principe actif antiviral y est présent en une quantité d'au moins 0,5 mg, d'au moins 2 mg, d'au moins 5 mg, d'au moins 10 mg, ou d'au moins 20 mg par unité de dosage.

2. Polysaccharide sulfaté destiné à être utilisé selon la revendication 1, dans lequel l'ingrédient actif antiviral comprend au moins un, éventuellement un mélange d'au moins deux desdits polysaccharides sulfatés.

3. Polysaccharide sulfaté destiné à être utilisé selon la revendication 1 ou 2, dans lequel
- ladite composition liquide ou semi-solide est choisie dans le groupe constitué d'une lotion pour la peau, d'une solution aqueuse pour la désinfection, d'une solution aqueuse pour l'inhalation, d'un spray ou d'une solution pulvérisable, de gouttes liquides et d'une solution de gargarisme;
- ladite composition semi-solide est choisie dans le groupe constitué d'un gel, d'une crème et d'une pommade; et
- ladite unité de dosage solide est choisie dans le groupe constitué d'un comprimé, d'une pastille, d'une dragée, d'une capsule et d'un chewing-gum.

4. Composition pharmaceutique ou médicament comprenant au moins un polysaccharide sulfaté choisi dans le groupe constitué de iota-carraghénane, kappa-carraghénane, lambda-carraghénane, sulfate de cellulose et sulfate de dextrane, en tant qu'ingrédient actif antiviral à utiliser dans l'application prophylactique ou thérapeutique contre une infection par le SRAS-CoV-2 chez un individu humain ou un animal non-humain, dans lequel
a) ladite composition ou ledit médicament se présente sous la forme d'un liquide, d'une mousse ou d'une préparation semi-solide et l'ingrédient actif antiviral y est présent à une concentration d'au moins 10 ug/ml, d'au moins 100 ug/ml ou d'au moins 400 ug/ml ;
ou bien, dans lequel
b) ladite composition ou ledit médicament se présente sous la forme d'une unité de dosage solide et le polymère sulfaté y est présent dans une quantité antivirale efficace d'au moins 0,5 mg, au moins 2 mg, au moins 5 mg, au moins 10 mg ou au moins 20 mg par unité de dosage.

5. Composition pharmaceutique ou médicament à utiliser selon la revendication 4, dans laquelle
ladite composition liquide ou semi-solide est choisie dans le groupe constitué d'une lotion pour la peau, d'une solution aqueuse pour la désinfection, d'une solution aqueuse pour l'inhalation, d'un spray ou d'une solution pulvérisable, de gouttes liquides et d'une solution de gargarisme;
dans laquelle ladite composition semi-solide est choisie dans le groupe constitué d'un gel, d'une crème et d'une pommade; et
dans laquelle ladite unité de dosage solide est choisie dans le groupe constitué d'un comprimé, d'une pastille, d'une dragée, d'une capsule et d'un chewing-gum.

6. La composition pharmaceutique ou le médicament à utiliser selon la revendication 4 ou 5, qui comprend un composant de carraghénane dans une quantité efficace antivirale, dans lequel ledit composant de carraghénane est soit iota-, kappa- ou lambda-carraghénane, soit un mélange d'au moins deux iota-, kappa- et lambda-carraghénane.

7. Composition pharmaceutique ou médicament à utiliser selon l'une des revendications 4 à 6, dans laquelle le lambda-carraghénane est purifié pour comprendre au moins 30 % en moles, de préférence 45 à 55 % en moles de sous-unités disaccharidiques répétitives de type lambda, par rapport au total des sous-unités disaccharidiques répétitives de type iota-, kappa-, lambda-, mu- et nu présentes dans le matériau lambda-carraghénane.

8. Composition pharmaceutique ou médicament à utiliser selon l'une des revendications 4 à 7, dans laquelle ladite composition ou ledit médicament comprend au moins un support et/ou un additif pharmaceutiquement acceptable, ledit additif étant de préférence choisi parmi le chlorure de sodium et le chlorure de potassium et étant présent dans la composition à raison de 1,5 % en poids ou moins, ou de 1,0 % en poids ou moins, ou de 0,6 % ou moins.

9. Composition pharmaceutique ou médicament à utiliser selon l'une des revendications 4 à 8, dans laquelle la composition sous forme liquide est fixée par enduction ou imprégnation sur une surface solide d'un article d'hygiène ou sanitaire.

10. Composition pharmaceutique ou médicament à utiliser selon la revendication 9, dans laquelle l'article hygiénique ou sanitaire est choisi dans le groupe comprenant un gant hygiénique ou sanitaire, un mouchoir ou papier hygiénique ou sanitaire, un coton-tige, un masque anti-poussière, un masque facial sanitaire et un masque facial médical.

11. Composition pharmaceutique ou médicament à utiliser selon la revendication 4, dans lequel l'individu humain est un patient à haut risque choisi dans le groupe constitué d'un patient atteint de BPCO, d'un patient asthmatique, d'une personne allergique, d'une personne dont le système immunitaire, cardiaque ou pulmonaire est affaibli, et d'un patient transplanté, ou dans lequel l'individu humain est un membre du personnel de santé dans les hôpitaux, les pharmacies et les cabinets médicaux, ou une personne qui entre professionnellement en contact étroit avec des individus humains ou des animaux non-humains infectés ou potentiellement infectés.
